# EUROPEAN PATENT SPECIFICATION

(11) **EP 2 883 546 B1**
(45) Date of publication and mention of the grant of the patent: **08.09.2021**
(21) Application number: 13822895.2
(22) Date of filing: 22.07.2013
(51) Int. Cl.: A61K 9/50, A61K 9/48, A61K 31/4422, A61K 31/4178, A61K 31/549, A61K 31/435, A61P 9/12

(54) **NEW DIFFERENTIAL-RELEASE PHARMACEUTICAL COMPOSITION CONTAINING THREE ACTIVE PRINCIPLES**
NEUE PHARMAZEUTISCHE ZUSAMMENSETZUNG MIT DREI WIRKSTOFFEN MIT DIFFERENZIELLER FREISETZUNG
NOUVELLE COMPOSITION PHARMACEUTIQUE À LIBÉRATION DIFFÉRENTIELLE CONTENANT TROIS PRINCIPES ACTIFS

(30) Priority: 23.07.2012 MX 2012008541
(43) Date of publication of application: 17.06.2015
(73) Proprietor: Landsteiner Scientific S.A. de C.V., 01900 Distrito Federal (MX)
(72) Inventor: AGAPITO PERALTA, Dario, C.P. 50266 Toluca Estado de México (MX); MARTÍNEZ REYES, Julián, C.P. 57180 Nezahualcoyoti Estado de México (MX); REYES MORENO, Carlos Alberto, C.P. 51205 Toluca Estado de México (MX)
(74) Representative: Covadonga Fernández-Vega Feijoo, Maria
(86) International application number: PCT/MX2013/000092
(87) International publication number: WO 2014/017897

(56) References cited:
- WO-A1-03/097045
- WO-A1-2009/134956
- US-A1- 2010 047 341
- US-A1- 2010 143 470
- Karunanidhi: "Simultaneous Analysis of Losartan Potassium, Amlodipine Besylate, and Hydrochlorothiazide in Bulk and in Tablets by High-Performance Thin Layer Chromatography with UV-Absorption Densitometry", J Anal Methods Chem, 8 April 2012 (2012-04-08), pages 1-10, XP55264827, Retrieved from the Internet: URL:http://www.ncbi.nlm.nih.gov/pmc/articl es/PMC3335319/ [retrieved on 2016-04-12]
- DATABASE WPI Derwent Publications Ltd., London, GB; AN 2010-A38512, XP055190227 & KR 2009 0 131 472 A (SAM CHUN DANG PHARM CO LTD) 29 December 2009
- None

## Description

### FIELD OF THE INVENTION

The present invention belongs to the field of controlled and differentiated-release pharmaceutical compositions of drugs useful in the treatment of heart conditions.

### BACKGROUND OF THE INVENTION

Drug combinations present certain advantages with respect to the possibility that they can be administered concomitantly in a single pharmaceutical form, thus abating the individual administration of each drug involved.

Several propositions have been developed today that make use of the drug combination to control a condition such as AH, specifically the use of losartan with amlodipine, as described in MULTILAYER PHARMACEUTICAL COMPOSITION COMPRISING TELMISARTAN AND AMLODIPINE (WO/2012/055941); COMBINED PREPARATION FOR THE TREATMENT OF CARDIOVASCULAR DISEASES BASED ON CHRONOTHERAPY THEORY (MXMX/a/2009/003489); PHARMACEUTICAL COMPOSITION COMPRISING AMLODIPINE AND LOSARTAN (MXMX/a/2008/016099).

However, the above compositions speak directly of the combination of losartan with amlodipine, and not of the incorporation of hydrochlorothiazide as renal protector.

Losartan, or losartan potassium, is an angiotensin-II receptor antagonist, indicated in the treatment of: arterial hypertension; heart failure, in combination with diuretics or digitalis (although the latter are increasingly less used in the treatment of heart failure); kidney disease in patients with type 2 diabetes with proteinuria and hypertension, as part of the antihypertensive treatment.

Amlodipine (or amlodipine manufactured by STADA) is a drug belonging to the group of dihydropyridines (see dihydropyridine), which acts as a long-action calcium channel blocker used in medicine as an antihypertensive and in the treatment of angina pectoris. Like other calcium blockers, amlodipine acts by relaxing the smooth muscle of the arterial wall, thus reducing peripheral resistance and, therefore, reducing arterial pressure. In angina pectoris its actions reduce the flow of blood to the heart muscle.

Amlodipine has been indicated for the treatment of arterial hypertension and in the prophylaxis of angina pectoris.

Amlodipine can be used in patients with heart failure; no increase in gross mortality rate has been noted in these cases with the use of amlodipine.¹ Amlodipine tends to improve left ventricular mass, although it is generally used in therapies that combine antihypertensives when the first-line drugs no longer have the desired effect on arterial tension.²

The collateral effects of amlodipine include:
- Frequently: Peripheral edema, muscle, stomach and head pain, dyspepsia, nausea in around 1 in 100 users.
- Sometimes: Vertigo, palpitations, mammary development in men (gynecomastia), erectile dysfunction, depression, insomnia and tachycardia in 1 in 1,000 users.
- Rarely: Erratic behavior, hepatitis, jaundice in 1 in 10,000 users.
- Very rarely: Hyperglycemia, tremor in 1 in 100,000 users.

Hydrochlorothiazide, sometimes abbreviated to HCT, HCTZ or HZT is a diuretic drug of first choice belonging to the thiazide group. It acts by inhibiting the capacity of the kidney to retain water, thereby increasing the amount of urine. This reduces the volume of blood, decreasing its return to the heart and thus cardiac output. In addition, through other mechanisms, it is thought to reduce peripheral vascular resistance. Hydrochlorothiazide is sold as a generic medicine and has several commercial names. The appropriate dose of hydrochlorothiazide may vary from patient to patient. It is generally used in arterial hypertension; congestive heart failure; edemas caused by heart, kidney or liver failure; renal diabetes insipidus; renal tubular acidosis; prevention of kidney stones and in idiopathic hypercalciuria.

Based on the above, having two useful drugs in the treatment of arterial hypertension with different action mechanisms (that is, while losartan potassium acts as an angiotensin-II receptor antagonist, amlodipine is a calcium channel blocker) is important because the two drugs do not compete for the same receptors or sites of action.

Calcium channel antagonists like amlodipine are powerful vasodilators that induce a reflex activation both in the nervous system and the RAAS, meaning that there is an increase in angiotensin-II values (by reducing the response to it by blocking the calcium influx required for its effects), as well as a negative sodium balance which enhances the antihypertensive effect of angiotensin-II receptor antagonists. In addition to not having a mechanism of action with common target receptors, the effect would be reflected in their therapeutic action, that is, the hypotensive effect. Furthermore, edema often presents during the administration of amlodipine; in this case the diuretics play a role protecting kidney function.

Clearly, having a medication that provides two or more useful drugs in the treatment of arterial hypertension with different mechanisms of action - losartan potassium and amlodipine with a synergistic effect, plus a diuretic to avoid problems associated with amlodipine - whether by sequential or differential release, would be highly advantageous compared to single-drug medications where the administration of each of the active principles is done separately and in one or two time intervals. In addition, a single dose is required to reach the therapeutic effect of the three drugs, which would guarantee adherence to the treatment.

A drug currently exists that combines losartan with hydrochlorothiazide; however, patent documents have not been published that describe amlodipine-losartan-hydrochlorothiazide combinations.

The Mexican application serial number 2008/16532 describes an amlodipine-hydrochlorothiazide combination; while Mexican patent 198140 discloses a composition containing losartan-hydrochlorothiazide.

MX/a/2011/006008 SOLID PHARMACEUTICAL COMPOSITION COMPRISING AMLODIPINE AND LOSARTAN describes a solid pharmaceutical composition for preventing or treating cardiovascular disorders comprising amlodipine and losartan as active ingredients, and a disintegrant which is a mixture of at least two components selected from the group consisting of sodium starch glycolate, crosscarmellose sodium, and crosspovidone, which exhibits a high and stable level of amlodipine and losartan dissolution rates. MX/a/2011/006009 SOLID PHARMACEUTICAL COMPOSITION COMPRISING AMLODIPINE AND LOSARTAN AND PROCESS FOR PRODUCING SAME refers to a pharmaceutical composition for preventing or treating cardiovascular disorders comprising amlodipine or an acceptable pharmaceutical salt thereof, which exhibits high amlodipine and losartan dissolution rates even under low pH conditions, and improved storage stability. MX/a/2011/006061 SOLID PHARMACEUTICAL COMPOSITION COMPRISING AMLODIPINE AND LOSARTAN WITH IMPROVED STABILITY reveals a solid pharmaceutical composition for preventing or treating cardiovascular diseases comprising granular forms of amlodipine and losartan which are separated from each other, and a stabilizing agent, which has improved storage stability due to minimized interaction between the amlodipine and losartan. And MX/a/2008/016532 SOLID DOSAGE FORMS OF VALSARTAN, AMLODIPINE AND HYDROCHLOROTHIAZIDE AND METHOD OF MAKING THE SAME describes the preparation of monolayer, bilayer and trilayer solid dosage forms of a combination of valsartan, amlodipine and hydrochlorothiazide.

Karunanidhi et al. discloses a tablet comprising hydrochlorothiazide, amlodipine and losartan (J Anal Methods Chem, published online 08-04-2012, pages 1-10).

As can be seen, the aforementioned documents do not anticipate in any way the composition subject of this application and which is described below.

### BRIEF DESCRIPTION OF THE DRAWINGS

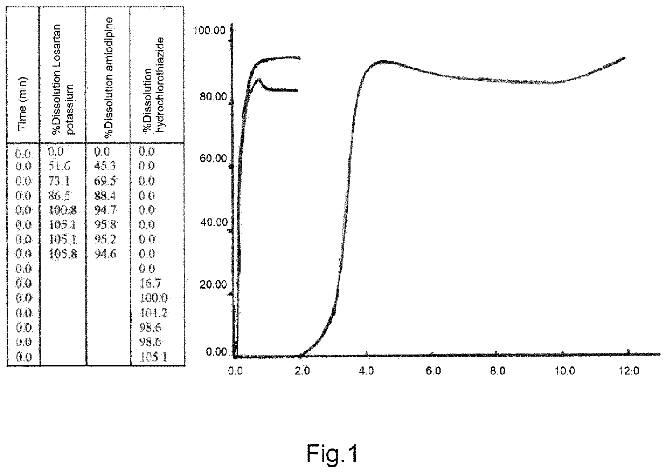

Figure 1 shows the dissolution profile of the composition subject of this invention.

### SUBJECT OF THE INVENTION

The aim of this invention is to offer a medication that requires a single dose to achieve the therapeutic effect of three drugs.

A second objective is to provide a multiparticulate product that can be placed inside a capsule or sachet.

A third objective is to offer the patient the comfort of a single dose of the medication once a day, with the advantage that, due to the sequential release of hydrochlorothiazide, the said dose can be taken before going to bed so that the diuretic takes effect the following morning.

An additional aim of this invention is to provide a manufacturing process for a product that combines three active principles, at least one with differential release.

A further objective of this invention is to provide a medication that gives the patient a sense of security and confidence by the immediate treatment of hypertension on the one hand, and the reduction of possible side effects (edemas), typical of amlodipine, on the other.

### SPECIFICATION OF THE INVENTION

Losartan potassium as such possesses a very brief half-life of approximately 2 hours; however, it has an active metabolite that is around 10 to 40 times more powerful whose half-life is between 6 and 9 hours, sufficient time to allow the action of the diuretic drug without any interference between the two. Furthermore, the scheduling of the therapeutic effect is tied to the drug absorption site, hence the effect between the active principles is related, enhancing the therapeutic effect.

The duration of administration of the differentiated or sequential drugs of the combination of the invention is achieved through a solid pharmaceutical form of sustained and sequential release of each drug.

The administration of the composition of this invention in triple-drug pellet form is controlled by an enteric barrier which enables the sequential release of losartan and amlodipine, and the later release of hydrochlorothiazide, while in the case of two-drug tablets, no.

The sequential release of losartan and amlodipine enables the hypertensive effect of one on the other to be synergistic; in addition, the combination contains a diuretic, hydrochlorothiazide, which increases the formation of sodium reabsorption channels which steadily improves the hypotensive effect due to this drug being administered around 4 hours (based on the chart in Figure 1) after the administration of the medication. This effect has a triple function: first, to increase the hypotensive effect; second, to avoid peripheral edemas that may result from the administration of amlodipine, and third, to encourage the biotransformation of the losartan and amlodipine avoiding the appearance of side effects of the two drugs. Moreover, if this medication is given during the night, the diuretic effect of the hydrochlorothiazide would present in the morning.

Support for the above statements can be found in Fogari R, Zoppi A, Derosa G, Mugellini A, Lazzari P, Rinaldi A, et al. Effect of valsartan addition to amlodipine on ankle oedema and subcutaneous tissue pressure in hypertensive patients. J Hum Hypertens. 2007;21:22O-4; Messerli FH. Vasodilatory edema: a common side effect of antihypertensive therapy. Curr Cardiol Reports. 2OO2;4:479-82; and Calhoun DA, Lacourcière T, Chiang YT, Glazer RD. Triple antihypertensive therapy with amlodipine, valsartan and hydrochlorothiazide: a randomized clinical trial.

The composition of the invention is defined in the claims. The composition comprises hydrochlorothiazide, amlodipine besylate, losartan potassium, a carrier or substrate, a composition of immediate-release water-soluble coating, an extended release composition, and a water-soluble total coating composition. The said composition is prepared in the form of pellets or microspheres packaged in a hard gelatin capsule.

The composition of the immediate release coating is water soluble, regardless of the pH, and allows immediate disintegration and rapid release of the active principle. The said composition comprises, expressed as a % of the total weight of the composition, 52% to 55% polyvinyl alcohol; 30% talc; between 14% and 16% polyethylene glycol, and 3% polysorbate 80.

The extended release composition forms a film and has plasticizer and stabilizer properties. The composition comprises a primary composition consisting of, expressed as % of total weight of the composition, 60% to 62% ethyl cellulose, 17% oleic acid, 14% to 16% ammonium hydroxide and 6% to 7% hydroxypropyl cellulose; and a secondary composition that is the immediate release composition, at a 9:1 weight ratio.

The composition of total coating, expressed as % of the total weight of the composition, is 40% polyvinyl alcohol, 20% to 22% polyethylene glycol, 18% to 20% titanium dioxide, 14% to 16% talc, and 5% to 7% of a pharmaceutically acceptable pigment.

The coatings described above can be obtained from various suppliers under commercial brands including, Opadry II, Opadry II Clear, Opadry II with pigment, Surelease Clear, and the like.

The new differential release pharmaceutical composition subject of this invention comprises 5% to 20% in weight of the composition of losartan potassium, 0.5% to 3% in weight of amlodipine, from 1% to 4% in weight of hydrochlorothiazide, 55% to 65% in weight of the cellulose sphere or sugar starch type carrier or substrate, 2% to 4% of a primary water-soluble immediate release coating, 7% to 9% of a second extended release coating, 2% to 4% of a third water-soluble immediate release coating, 4% to 6% of a fourth water-soluble immediate release coating and 6% to 8% of a layer of immediate dissolution water-soluble total coating.

The composition of this invention has the form of pellets or microspheres, which are poured into a hard gelatin capsule for distribution.

An example of the composition of this invention is shown in Table 1.

**TABLE 1**

| **Component** | | **mg** |
|---|---|---|
| Sugar-starch spheres | | 308.8 |
| Hydrochlorothiazide | | 12.5 |
| Opadry II Clear 85F 19250* | | 11.3 |
| Surelease Clear E-7-19050** | | 36.7 |
| Opadry II Clear 85F 19250* | | |
| Amlodipine besylate | | 6.9 |
| Opadry II Clear 85F 19250* | | 25 |
| Losartan potassium | | 50 |
| Opadry II Clear 85F 19250* | | |
| Opadry II Blue 85F 90631 *** | | 40 |

| | | |
|---|---|---|
| ^{∗} Contains 52.6% polyvinyl alcohol; 30% talc; 14.74% polyethylene glycol and 3% polysorbate. ^{∗∗} Contains 61.23% ethylene cellulose; 17% oleic acid; 14.96% ammonium hydroxide and 6.8% hydroxypropyl cellulose. ^{∗∗∗} Contains 66.66% polyvinyl alcohol; 30% talc; 33% polyethylene glycol and 3% polysorbate. | | |

The composition in Table 1 is manufactured based on the following process:
The sugar-starch spheres are loaded into a fluid bed apparatus with the necessary fixtures to perform the coating of the microspheres. It is heated for at least 15 minutes to a temperature between 60°C and 70°C.

The first coating dispersion is prepared separately placing 960g of purified water in a container and adding Opadry II Clear 85F19250 under agitation. Once the mixture is uniform, hydrochlorothiazide is added, continuing agitation until fully incorporated.

The above dispersion is added over the microsphere substrate, maintaining the inlet temperature at between 45°C and 55°C.

The second coating dispersion is prepared separately placing 760g of purified water in a container and then adding Opadry II Clear 85F19250. Once incorporated, Surelease Clear E-7-19250 is added.

The above mixture is added to the substrate and first coating mixture maintaining the inlet temperature at between 55°C and 65°C.

A third coating dispersion is prepared placing 680g of purified water in a container and incorporating Opadry II Clear 85F19250. Once incorporated, amlodipine besylate is added.

The coating is applied maintaining the inlet temperature at between 50°C and 60°C.

The fourth coating layer is prepared placing 2400g of purified water in an appropriate container under constant agitation and incorporating Opadry II Clear 85F19250. Once incorporated, 400g of losartan potassium is added.

The coating is applied maintaining the temperature at between 50°C and 60°C.

The fifth coating layer is prepared placing 1600g of purified water in an appropriate container under constant agitation and adding Opadry II Blue 85F90631 until completely uniform.

The coating is applied maintaining the temperature at between 50°C and 60°C.

Once the impregnation process is finished, the pellets are unloaded from the bowl. 500mg of pellets is filled into Size 0 natural/natural capsules.

The composition obtained from Table 1 shows a dissolution profile represented in Figure 1, where it can be seen that losartan and amlodipine are released immediately, as required in the treatment of hypertension, and hydrochlorothiazide is released up to six hours later, which is when it imparts its therapeutic effect aimed at reducing symptoms of edema in the patient.

These compositions are given by way of example, but in no way limit the scope of the description of this invention.

## Claims

1. New differential release pharmaceutical composition in the form of pellets or microspheres comprising:
- losartan potassium in amount of 5% to 20% by weight of the composition;
- amlodipine in amount of 0.5% to 3% by weight of the composition;
- hydrochlorothiazide in amount of 1% to 4% by weight of the composition;
- sugar-starch spheres carrier or substrate in amount of 55% to 65% by weight of composition; and
- a first coating in amount of 2% to 4% by weight of the composition, wherein the first coating is an immediate release coating that it is water soluble regardless of the pH and its composition is, expressed as % of the total weight, 52% to 55% polyvinyl alcohol; 30% talc; between 14% and 16% polyethylene glycol and 3% polysorbate 80; and comprising hydrochlorothiazide;
- a second coating in amount of 7% to 9% by weight of the composition, wherein the second coating is an extended release coating that it forms a film, having plasticizer and stabilizer characteristics and it has a primary composition consisting of, expressed as % of the total weight, 60% to 62% ethyl cellulose, 17% oleic acid, 14% to 16% ammonium hydroxide and 6% to 7% hydroxypropyl cellulose; a secondary composition that is the composition of the first coating; and wherein the weight ratio of the primary composition to the second is 9:1;
- a third coating in amount of 2% to 4% by weight of the composition, wherein the third coating is an immediate release coating that it is water soluble regardless of the pH and its composition is, expressed as % of the total weight, 52% to 55% polyvinyl alcohol; 30% talc; between 14% and 16% polyethylene glycol and 3% polysorbate 80; and comprising amlodipine besylate;
- a fourth coating in amount of 4% to 6% by weight of the composition, wherein the fourth coating is an immediate release coating that it is water soluble regardless of the pH and its composition is, expressed as % of the total weight, 52% to 55% polyvinyl alcohol; 30% talc; between 14% and 16% polyethylene glycol and 3% polysorbate 80; and comprising losartan potassium; and
- a fifth coating in amount of 6% to 8% by weight of the composition, wherein the fifth coating is an immediate dissolution water soluble layer that its composition, expressed as % of the total weight, comprises 40% polyvinyl alcohol; 20% to 22% polyethylene glycol; 18% to 20% titanium dioxide; 14% to 16% talc, and 5% to 7% of a pharmaceutically acceptable pigment

2. New differential release pharmaceutical compositions, according to Claim 1, in which the form of pellets or microspheres is **characterized in that** are poured into a hard gelatin capsule for distribution.

3. A process for the manufacture of the composition according to Claim 1, in which the coatings are prepared separately, the said process is **characterized in that** it comprises the following stages:
A. preparing the substrate of sugar-starch spheres by heating it for at least 15 minutes to a temperature between 60°C and 70°C;
B. preparing the first coating dispersion, adding hydrochlorothiazide, and agitating untill fully incorporated;
C. adding the above dispersion over the carrier or substrate of sugar-starch spheres, and maintaining the inlet temperature at between 45°C and 55°C;
D. adding the second coating dispersion over the mixture of the first dispersion and the substrate maintaining inlet temperature at between 55°C and 65°C;
E. incorporating the third coating, and once incorporated, adding amlodipine besylate, and maintaining the inlet temperature at between 50°C and 60°C;
F. incorporating the fourth coating under constant agitation, and once incorporated adding losartan potassium, and maintaining the temperature at between 50°C and 60°C; and
G. adding the fifth coating until completely uniform, and maintaining the temperature at between 50°C and 60°C.

## Patentansprüche

1. Neue pharmazeutische Zusammensetzung mit differenzieller Freisetzung in Form von Pellets oder Mikrokügelchen, umfassend:
- Losartan-Kalium in einer Menge von 5 Gew.-% bis 20 Gew.-% der Zusammensetzung;
- Amlodipin in einer Menge von 0,5 Gew.-% bis 3 Gew.-% der Zusammensetzung;
- Hydrochlorothiazid in einer Menge von 1 Gew.-% bis 4 Gew.-% der Zusammensetzung;
- einen Träger oder ein Substrat aus Zucker-Stärke-Kugeln in einer Menge von 55 Gew.-% bis 65 Gew.-% der Zusammensetzung; und
- eine erste Beschichtung in einer Menge von 2 Gew.-% bis 4 Gew.-% der Zusammensetzung, wobei die erste Beschichtung eine Beschichtung mit sofortiger Freisetzung ist, welche unabhängig vom pH-Wert wasserlöslich ist und deren Zusammensetzung, als % des Gesamtgewichts ausgedrückt, 52% bis 55% Polyvinylalkohol; 30% Talk; zwischen 14% und 16% Polyethylenglykol und 3% Polysorbat 80 ist; und umfassend Hydrochlorothiazid;
- eine zweite Beschichtung in einer Menge von 7 Gew.-% bis 9 Gew.-% der Zusammensetzung, wobei die zweite Beschichtung eine Beschichtung mit verzögerter Freisetzung ist, welche einen Film bildet, aufweisend Weichmacher- und Stabilisatoreigenschaften und mit einer primären Zusammensetzung bestehend aus, als % des Gesamtgewichts ausgedrückt, 60% bis 62% Ethylcellulose, 17% Ölsäure, 14% bis 16% Ammoniumhydroxid und 6% bis 7% Hydroxypropylcellulose; mit einer sekundären Zusammensetzung, welche die Zusammensetzung der ersten Beschichtung ist; und wobei das Gewichtsverhältnis der primären Zusammensetzung in Bezug auf die zweite 9:1 ist;
- eine dritte Beschichtung in einer Menge von 2 Gew.-% bis 4 Gew.-% der Zusammensetzung, wobei die dritte Beschichtung eine Beschichtung mit sofortiger Freisetzung ist, welche unabhängig vom pH-Wert wasserlöslich ist und deren Zusammensetzung, als % des Gesamtgewichts ausgedrückt, 52% bis 55% Polyvinylalkohol; 30% Talk; zwischen 14% und 16% Polyethylenglykol und 3% Polysorbat 80 ist; und umfassend Amlodipinbesylat;
- eine vierte Beschichtung in einer Menge von 4 Gew.-% bis 6 Gew.-% der Zusammensetzung, wobei die vierte Beschichtung eine Beschichtung mit sofortiger Freisetzung ist, welche unabhängig vom pH-Wert wasserlöslich ist und deren Zusammensetzung, als % des Gesamtgewichts ausgedrückt, 52% bis 55% Polyvinylalkohol; 30% Talk; zwischen 14% und 16% Polyethylenglykol und 3% Polysorbat 80 ist; und umfassend Losartan-Kalium;
- eine fünfte Beschichtung in einer Menge von 6 Gew.-% bis 8 Gew.-% der Zusammensetzung, wobei die fünfte Beschichtung eine wasserlösliche Schicht mit sofortiger Auflösung ist, dessen Zusammensetzung, als % des Gesamtgewichts ausgedrückt, 40% Polyvinylalkohol; 20% bis 22% Polyethylenglykol; 18% bis 20% Titandioxid; 14% bis 16% Talk, und 5% bis 7% eines pharmazeutisch akzeptablen Pigments umfasst.

2. Neue pharmazeutische Zusammensetzungen mit differenzieller Freisetzung nach Anspruch 1, in welchen die Form von Pellets oder Mikrokügelchen **dadurch gekennzeichnet ist, dass** sie in eine Hartgelatinekapsel für deren Verteilung eingefüllt werden.

3. Verfahren zur Herstellung der Zusammensetzung nach Anspruch 1, in welchem die Beschichtungen separat zubereitet werden, wobei das genannte Verfahren **dadurch gekennzeichnet ist, dass** es die folgenden Schritte umfasst:
A. das Zubereiten des Substrats aus Zucker-Stärke-Kugeln, indem es mindestens 15 Minuten lang bis zu einer Temperatur zwischen 60°C und 70°C erwärmt wird;
B. das Zubereiten der ersten Beschichtungsdispersion, das Hinzufügen von Hydrochlorothiazid und das Rühren, bis es vollständig integriert ist;
C. das Hinzufügen der zuvor erwähnten Dispersion über dem Träger oder Substrat aus Zucker-Stärke-Kugeln, und das Aufrechterhalten der Eintrittstemperatur bei zwischen 45°C und 55°C;
D. das Hinzufügen der zweiten Beschichtungsdispersion über der Mischung aus der ersten Dispersion und dem Substrat, unter Aufrechterhaltung der Eintrittstemperatur bei zwischen 55°C und 65°C;
E. das Integrieren der dritten Beschichtung und, sobald sie integriert ist, das Hinzufügen von Amlodipinbesylat, und das Aufrechterhalten der Eintrittstemperatur bei zwischen 50°C und 60°C;
F. das Integrieren der vierten Beschichtung unter konstantem Rühren und, sobald sie integriert ist, das Hinzufügen von Losartan-Kalium und das Aufrechterhalten der Temperatur bei zwischen 50°C und 60°C; und
G. das Hinzufügen der fünften Beschichtung, bis sie vollständig gleichmäßig ist, und das Aufrechterhalten der Temperatur bei zwischen 50°C und 60°C.

## Revendications

1. Nouvelle composition pharmaceutique à libération différentielle sous forme de granules ou de microsphères qui comprend :
- losartan potassium dans une quantité de 5% à 20% en poids de la composition ;
- amlodipine dans une quantité de 0,5% à 3% en poids de la composition ;
- hydrochlorothiazide dans une quantité de 1% à 4% en poids de la composition ;
- porteur ou substrat de sphères de sucre-amidon dans une quantité de 55% à 65% en poids de la composition ; et
- un premier enrobage dans une quantité de 2% à 4% en poids de la composition, dans laquelle le premier enrobage est un enrobage à libération immédiate qui est soluble dans l'eau indépendamment du pH et sa composition est, exprimée en % du poids total, de 52% à 55% de poly(alcool vinylique) ; 30% de talc ; entre 14% et 16% de polyéthylèneglycol et 3% de polysorbate 80 ; et qui comprend de l'hydrochlorothiazide ;
- un second enrobage dans une quantité de 7% à 9% en poids de la composition, dans laquelle le second enrobage est un enrobage à libération prolongée qui forme une pellicule, qui a des caractéristiques plastifiantes et stabilisatrices et a une composition principale qui consiste en, exprimée en % du poids total, de 60% à 62% d'éthylcellulose, 17% d'acide oléique, de 14% à 16% d'hydroxyde d'ammonium et de 6% à 7% d'hydroxypropylcellulose ; une composition secondaire qui est la composition du premier enrobage ; et dans laquelle la raison en poids de la composition principale par rapport à la seconde est de 9:1 ;
- un troisième enrobage dans une quantité de 2% à 4% en poids de la composition, dans laquelle le troisième enrobage est un enrobage à libération immédiate qui est soluble dans l'eau indépendamment du pH et sa composition est, exprimée en % du poids total, de 52% à 55% de poly(alcool vinylique) ; 30% de talc; entre 14% et 16% de polyéthylèneglycol et 3% de polysorbate 80 ; et qui comprend du bésylate d'amlodipine ;
- un quatrième enrobage dans une quantité de 4% à 6% en poids de la composition, dans laquelle le quatrième enrobage est un enrobage à libération immédiate qui est soluble dans l'eau indépendamment du pH et sa composition est, exprimée en % du poids total, de 52% à 55% de poly(alcool vinylique) ; 30% de talc ; entre 14% à 16% de polyéthylèneglycol et 3% de polysorbate 80 ; et qui comprend du losartan potassium ; et
- un cinquième enrobage dans une quantité de 6% à 8% en poids de la composition, dans laquelle le cinquième enrobage est une couche soluble dans l'eau, à dissolution immédiate, dont la composition, exprimée en % du poids total, comprend 40% de poly(alcool vinylique) ; de 20% à 22% de polyéthylèneglycol ; de 18% à 20% de dioxyde de titane ; de 14% à 16% de talc ; et de 5% à 7% d'un pigment pharmaceutiquement acceptable.

2. Nouvelles compositions pharmaceutiques à libération différentielle selon la revendication 1, dans lesquelles la forme des granules ou des microsphères est **caractérisée en ce qu'**elles sont versées dans une capsule de gélatine dure pour leur distribution.

3. Procédé pour la fabrication de la composition selon la revendication 1, dans lequel les enrobages sont préparés séparément, ledit procédé est **caractérisé en ce qu'**il comprend les étapes suivantes :
A. préparer le substrat de sphères de sucre-amidon en les chauffant pendant au moins 15 minutes jusqu'à une température d'entre 60°C et 70°C ;
B. préparer la dispersion du premier enrobage, ajouter de l'hydrochlorothiazide et agiter jusqu'à ce qu'il soit complètement incorporé ;
C. ajouter la dispersion antérieure sur le porteur ou substrat de sphères de sucre-amidon, et maintenir la température d'entrée à entre 45°C et 55°C ;
D. ajouter la dispersion du second enrobage sur le mélange de la première dispersion et le substrat et maintenir la température d'entrée à entre 55°C et 65°C ;
E. incorporer le troisième enrobage, et une fois incorporé, ajouter du bésylate d'amlodipine, et maintenir la température d'entrée à entre 50°C et 60°C ;
F. incorporer le quatrième enrobage avec agitation constante, et une fois incorporé, ajouter du losartan potassium, et maintenir la température à entre 50°C et 60°C ; et
G. ajoutez le cinquième enrobage jusqu'à ce qu'il soit complètement uniforme, et maintenir la température à entre 50°C et 60°C.
